# FASCICULE DE BREVET EUROPEEN

(11) **EP 4 043 065 B1**
(45) Date de publication et mention de la délivrance du brevet: **12.08.2026**
(21) Numéro de dépôt: 22160463.0
(22) Date de dépôt: 02.10.2012
(51) Int. Cl.: A61N 1/05, A61N 1/372

(54) **MICROSONDE DE DÉTECTION/STIMULATION, IMPLANTABLE DANS DES RÉSEAUX VEINEUX, ARTÉRIELS OU LYMPHATIQUES**
MIKROSONDE ZUR DETEKTION/STIMULATION, DIE IN VENEN-, ARTERIEN- ODER LYMPHSYSTEM-NETZE IMPLANTIERT WERDEN KANN
DETECTION/STIMULATION MICROPROBE IMPLANTABLE IN VENOUS, ARTERIAL OR LYMPHATIC SYSTEMS

(30) Priorité: 14.10.2011 FR 1159321
(43) Date de publication de la demande: 17.08.2022
(62) Numéro(s) de document de la (des) demande(s) initiale(s) en application de l'article 76 CBE: 12187052.1 / 2 581 107
(73) Titulaire: Sorin CRM SAS, 92140 Clamart (FR)
(72) Inventeur: OLLIVIER, Jean-Francois, 91190 Gif sur Yvette (FR); SHAN, Nicolas, 92160 Antony (FR); D'HIVER, Philippe, 92320 Chatillon (FR)
(74) Mandataire: Gulde & Partner

(56) Documents cités:
- EP-A1- 2 145 648
- EP-A1- 2 455 131
- WO-A1-00/54833
- WO-A2-2005/081681
- US-A- 5 246 014
- US-A1- 2003 139 794
- US-A1- 2006 247 751
- US-A1- 2007 225 784
- US-A1- 2009 157 136
- US-A1- 2010 211 147

## Description

L'invention concerne de façon générale les "dispositifs médicaux implantables actifs" tels que définis par la directive 90/385/CEE du 20 juin 1990 du Conseil des communautés européennes.

Cette définition inclut en particulier les implants cardiaques chargés de surveiller l'activité cardiaque et de générer des impulsions de stimulation, de défibrillation et/ou de resynchronisation en cas de trouble du rythme détecté par l'appareil. Elle inclut aussi les appareils neurologiques, les implants cochléaires, les pompes de diffusion de substances médicales, les capteurs biologiques implantés, etc.

Ces dispositifs comportent un boîtier généralement désigné "générateur", raccordé électriquement et mécaniquement à une ou plusieurs "sondes" intracorporelles munie(s) d'électrodes destinées à venir en contact avec les tissus sur lesquels on souhaite appliquer des impulsions de stimulation et/ou recueillir un signal électrique : myocarde, nerf, muscle, ...

La présente invention concerne plus précisément une microsonde de détection/stimulation destinée à être implantée dans des réseaux veineux, artériels ou lymphatiques.

Le principe actuel de stimulation électrique des tissus est basé sur un dispositif, appelé généralement "sonde", qui est un objet implanté au travers de divers vaisseaux, veineux, artériels ou lymphatiques notamment, et dont la fonction est de transmettre un signal électrique au tissu-cible tout en garantissant les propriétés suivantes :
- facilité d'implantation par le médecin dans un réseau de vaisseaux du patient, et en particulier facilité : à faire progresser la sonde dans les vaisseaux par poussée, à faire suivre à la sonde des trajets tortueux et passer des embranchements, et à transmettre des couples ;
- visibilité aux rayons X afin de permettre au médecin une navigation aisée à travers les vaisseaux du réseau sous radioscopie X ;
- atraumaticité de la sonde au niveau des veines, ce qui nécessite une grande souplesse de structure et l'absence de transition rigide ou d'angle vif ;
- aptitude à transmettre un signal électrique aux tissus et à faire des mesures électriques monopolaires ou multipolaires de manière stable ;
- biocompatibilité avec les tissus vivants pour une implantation à long terme ;
- capacité à se connecter à un appareil implantable source de transmission du signal ;
- aptitude à la stérilisation (rayons gamma, température ...) sans subir de dommages ;
- biostabilité, en particulier résistance à la corrosion dans le milieu vivant et résistance à la sollicitation mécanique en fatigue liée aux mouvements du patient et des organes ;
- compatibilité avec l'imagerie IRM, particulièrement importante en neurologie.

L'architecture actuelle des sondes répondant à ces besoins peut se résumer à une structure généralement creuse pour permettre le passage d'un mandrin ou d'un fil-guide, et comprenant des composants de type câbles conducteurs isolés, reliés à des électrodes mécaniques pour assurer la conductivité électrique, la radio-opacité ...

Il s'agit donc de sondes nécessitant un assemblage complexe d'un nombre important de pièces, de fils et d'isolants associés, créant des risques non négligeables de rupture compte tenu des contraintes mécaniques à long terme auxquelles elles sont exposées.

Des exemples de telles sondes sont donnés dans les EP 2145648 A1, US 6 192 280 A et US 7 047 082 A, ou encore dans le US 5 246 014 A..

Parmi les difficultés rencontrées, on peut citer la gestion des gradients de raideur liés aux pièces mécaniques utilisées, qui affectent fortement les propriétés d'implantabilité et de résistance mécanique sur le long terme (fatigue).

De plus, afin d'étanchéifier la lumière interne des sondes, dont l'entrée de sang dégraderait les performances à la pose et à long terme, on utilise des valves et autres dispositifs complexes présentant des risques associés importants.

D'autres difficultés peuvent également se poser en termes de fatigue des assemblages. En effet, toute zone de transition de rigidité est susceptible d'induire des risques de fatigue, des difficultés à stériliser du fait de la présence de zones d'accès difficile, et des problèmes de tenue des jonctions de conducteurs au niveau de la liaison avec les électrodes et le connecteur.

Par ailleurs, la tendance clinique dans le domaine des sondes implantables est d'en réduire la taille afin de les rendre moins invasives et plus faciles à manipuler à travers les vaisseaux.

La taille actuelle des sondes implantables est typiquement de l'ordre de 4 à 6 French (1,33 à 2 mm) dans leur partie active, c'est-à-dire la partie la plus distale portant la ou les électrodes - même si le corps de sonde, dans la partie moins distale, utilise des conducteurs de plus faible diamètre, comme par exemple dans le US 5 246 014 A précité qui, au niveau du corps de sonde, comprend certes un conducteur dont le diamètre ne dépasse pas 1 French (0,33 mm), mais dont le diamètre hors-tout de la partie distale active, à l'endroit de la vis d'ancrage, est de plusieurs French.

Cependant, il est clair que la réduction de la taille des sondes augmenterait leur complexité et imposerait des contraintes techniques génératrices de risques.

Pourtant, une telle réduction, à moins de 2 French (0,66 mm) par exemple, ouvrirait des perspectives d'applications médicales dans des domaines variés allant de la cardiologie à la neurologie en présence d'un réseau veineux, artériel ou même lymphatique, comme le réseau veineux cérébral ou le réseau veineux du sinus coronaire.

Aujourd'hui, la technologie de stimulation électrique a permis des avancées importantes dans le domaine de la neuromodulation, qui consiste à stimuler des zones-cibles du cerveau pour le traitement de la maladie de Parkinson, l'épilepsie et autres maladies neurologiques.

On pourrait donc imaginer parvenir avec ce type de technologie à traiter de nouvelles régions difficilement atteignables aujourd'hui, ceci au moyen de sondes de stimulation de petite taille, ou "microsondes", présentant une grande robustesse afin de garantir la biostabilité à long terme. Une telle technique permettrait une approche moins invasive de ces traitements et surtout une efficacité supérieure des traitements administrés.

Il serait possible de connecter une ou plusieurs microsondes à travers le réseau de vaisseaux considéré jusqu'à leur localisation-cible. Leur mise en place pourrait être effectuée, du fait de leur taille réduite, par des dispositifs de guidage utilisés aujourd'hui en neuroradiologie interventionnelle pour la libération de ressorts (*coils*) lors du traitement des anévrismes intracrâniens.

Aussi, le but de la présente invention est de proposer une telle "microsonde" de petite taille qui serait en conformité avec les propriétés générales des sondes implantables telles qu'elles ont été énumérées plus haut, tout en en réduisant la complexité et, de ce fait, le coût final.

La taille de cette microsonde devrait notamment permettre d'atteindre des veinules de très petites dimensions, inaccessibles aujourd'hui avec des dispositifs de taille supérieure. La microsonde de l'invention devrait également faciliter sensiblement la navigabilité dans les réseaux veineux, artériels ou lymphatiques du fait de sa souplesse, amplifiée par ses petites dimensions.

Conformément à l'invention, ce but est atteint grâce une sonde selon la revendication 1.

Ainsi, on comprend qu'avec un diamètre ne dépassant pas 0,50 mm, le câble de cœur constituant le microcâble de la microsonde selon l'invention présente une grande flexibilité, favorable à sa manipulation par le médecin, notamment au cours de son implantation lorsqu'il s'agit par exemple de l'introduire dans des réseaux de vaisseaux avec de fortes tortuosités et de nombreux embranchements et d'éviter des traumatismes que pourraient engendrer des sondes beaucoup plus rigides, incompatibles avec les tissus.

D'autre part, le choix d'une structure multifilaire toronnée composée de brins très fins, de diamètre unitaire d'au plus 40 µm, de préférence compris entre 20 et 40 µm, permet d'augmenter la résistance à la fatigue mécanique du câble de cœur due aux mouvements du patient et des organes, sachant que la limite de rupture en flexion d'un fil est sensiblement inversement proportionnelle à son diamètre. De manière à renforcer cette importante propriété de biostabilité, il y a avantage à ce que les brins eux-mêmes soient constitués en un matériau structurant dont la résistance à la fatigue intrinsèque est élevée, comme par exemple les matériaux cités plus haut, c'est-à-dire les aciers inox, les alliages de cobalt, le titane et l'alliage NiTi connu notamment sous le nom de nitinol. A ces métaux chargés d'assurer les qualités mécaniques du câble de cœur s'ajoute un matériau radio-opaque destiné à rendre la microsonde visible aux rayons X lors de sa mise en place par le médecin. Le matériau radio-opaque peut être choisi parmi le tantale (Ta), le tungstène (W), l'iridium (Ir), le platine (Pt), l'or (Au) et leurs alliages.

Concrètement, l'invention prévoit que la structure composite ainsi obtenue du câble de cœur peut être réalisée par des brins composites constitués, au moins, d'un matériau structurant et d'un matériau radio-opaque, ou par des brins constitués de matériau structurant et des brins constitués de matériau radio-opaque. La pluralité de brins du toron comprend avantageusement entre 15 et 300 brins.

Pour réaliser le contact électrique avec les tissus et transmettre le signal électrique, l'invention propose donc une solution qui consiste à utiliser le câble de cœur lui-même pour former les électrodes de la microsonde en ménageant des zones dénudées dans une couche d'isolement entourant le câble. L'invention recommande que la couche d'isolement, de préférence en polymère fluoré, ne dépasse pas en épaisseur 30% du diamètre du câble de cœur, ceci dans le but d'éviter l'effet d'escalier en bordure des électrodes qui pourrait nuire au contact électrique avec les tissus.

Enfin, les moyens de dégressivité de rigidité (c'est-à-dire qui procurent une rigidité décroissante de la partie proximale vers la partie distale) prévus par l'invention facilitent l'implantation de la microsonde grâce à la faculté qui lui est donnée de pouvoir être poussée à l'intérieur des vaisseaux. Comme on le verra en détail plus loin, les moyens de dégressivité de rigidité peuvent être réalisés, conformément à l'invention, par un empilement étagé de tubes emboîtés les uns dans les autres, ou par une succession de tubes isodiamètre de rigidité croissante.

La microsonde, une fois en place, est stabilisée en position par préformage, en S ou en spirale à trois dimensions, lequel assure également un contact électrique permanent des électrodes avec les tissus. Avantageusement, la microsonde comprend en outre des moyens de renforcement local.

Afin de limiter l'échauffement du câble de cœur par effet de peau lors d'une imagerie par IRM, l'invention recommande que les brins comprennent une couche extérieure en matériau à faible susceptibilité magnétique, inférieure à 2 000.10⁻¹².m³.mole⁻¹.

Le matériau à faible susceptibilité magnétique peut être, au choix, le tantale (Ta), le titane (Ti), le rhodium (Rh), le molybdène (Mo), le tungstène (W), le palladium (Pd), l'or (Au) et leurs alliages.

On va maintenant décrire un exemple de mise en œuvre du dispositif de l'invention, en référence aux dessins annexés où les mêmes références numériques désignent d'une figure à l'autre des éléments identiques ou fonctionnellement semblables.
Les Figures 1a à 1d sont des vues en coupe de brins constitués d'un matériau structurant et d'un matériau radio-opaque.
Les Figures 2a à 2f sont des vues en coupe de câbles de cœur formés de brins montrés sur les Figures 1a à 1d.
La Figure 3 est une vue en coupe d'un câble de cœur formé de brins structurants et de brins radio-opaques.
Les Figures 4a à 4d sont des vues de côté de préformes de microsondes selon l'invention.
La Figure 5 est une vue en perspective d'un microcâble présentant une zone totalement dénudée de la couche d'isolement.
La Figure 6 est une vue en perspective d'un microcâble présentant une zone partiellement dénudée de la couche d'isolement.
Les Figures 7a et 7b sont des vues en coupe de microcâbles présentant des zones de couche d'isolement partiellement dénudées.
La Figure 8 est une vue de côté en coupe d'une microsonde présentant un empilement de tubes de gradient de rigidité.
La Figure 9 est une vue en perspective d'une microsonde munie d'un dispositif de renforcement local.
La Figure 10a est une vue de côté d'un premier mode de réalisation d'une microsonde conforme à l'invention.
La Figure 10b est une vue en coupe de la section distale de la microsonde de la Figure 10a.
La Figure 10c est une vue en coupe d'un brin unitaire du câble de cœur de la microsonde de la Figure 10b.
La Figure 10d est une vue en perspective de la microsonde de la Figure 10a munie d'un connecteur IS-1.
La Figure 10e est une vue en perspective montrant une implantation de la microsonde de la Figure 10a dans les veines coronaires.
La Figure 11a est une vue en coupe d'un deuxième mode de réalisation d'une microsonde conforme à l'invention.
La Figure 11b est une vue en coupe d'un brin unitaire du câble de cœur de la microsonde de la Figure 11a.
Les Figures 11c et 11d sont des vues en perspective montrant des implantations de la microsonde de la Figure 11a dans une cavité du cœur.
La Figure 12a est une vue en coupe d'un troisième mode de réalisation d'une microsonde conforme à l'invention.
La Figure 12b est une vue en coupe d'un brin unitaire du câble de cœur de la microsonde de la Figure 12a.
La Figure 12c est une vue en coupe d'un exemple d'implantation de la microsonde de la Figure 12a dans les tissus cérébraux.

Les sondes concernées par l'invention sont des microsondes de stimulation destinées à être implantées dans des réseaux veineux, artériels ou lymphatiques, et dont le diamètre ne dépasse pas 2 French (0,66 mm). Elles sont constituées dans leur partie active, distale, par un microcâble formé d'un câble de cœur conducteur entouré partiellement d'une couche d'isolement définissant au moins une électrode de stimulation.

La durée de vie est un paramètre fondamental qui doit être absolument pris en compte lors de la conception de tout dispositif médical, en particulier les microsondes de stimulation, objets de l'invention. En effet, les battements du cœur et le mouvement des organes induisent sur ce type de dispositifs des déformations de flexion qui doivent être parfaitement maitrisées.

D'une manière générale, pour un fil cylindrique de diamètre d, la déformation en flexion peut être caractérisée par le rapport ε *= d*/*D* où D représente le diamètre de la courbure imposée au fil par la contrainte de flexion. Cette contrainte, par exemple liée au battement cardiaque, pourra être subie par le brin sur 400.10⁶ cycles sur une période de 10 ans, engendrant une fatigue du matériau pouvant entraîner sa rupture et limiter sa durée de vie.

C'est ainsi qu'une microsonde de stimulation par le réseau veineux, par exemple, peut être le siège de déformations de courbure supérieures à une sonde normale dans la mesure où elle doit suivre les déformations des veines, ce qui provoque une contrainte plus importante et rend sa résistance à la fatigue plus contraignante.

Pour augmenter la limite de rupture à la fatigue des microsondes, il y a donc avantage à adopter pour le câble de cœur une structure multifilaire sous la forme d'un toron d'une pluralité de brins conducteurs de faible diamètre *d.* La réduction en diamètre des brins unitaires permet en effet de réduire la contrainte appliquée à chacun d'entre eux et donc d'augmenter les performances en fatigue de la structure du toron. Pour un matériau donné, il est possible de définir une déformation maximale *c_{Max}* correspondant à sa limite de résistance à la fatigue pour un nombre de cycles de déformation égal par exemple à 100.10⁶.

Le choix d'un matériau constituant l'armature du câble de cœur, qu'on appellera "matériau structurant", doit répondre à plusieurs critères.

Il doit notamment faire partie des matériaux dont les propriétés mécaniques sont connus pour des applications implantables à long terme et présenter une déformation maximale ε*_{Max}* supérieure à la déformation qu'un brin est susceptible de subir, tout en restant compatible avec la faisabilité technique et le coût d'un brin de très faible diamètre.

A titre d'exemple, pour un alliage de cobalt de type MP35N ayant une déformation maximale ε_{Max} de 5.10⁻³ à 100.10⁶ cycles et pour un diamètre de courbure *D* de 7 mm, le diamètre du brin unitaire devra être inférieur à 35 µm. Un brin de 20 µm résistera donc facilement à cette sollicitation, tandis qu'un brin de 40 µm risquera d'engendrer une rupture avant d'atteindre les 100.10⁶ cycles. A noter que les alliages NiTi présentent de déformation maximale ε*_{Max}* supérieure, de 5 à 9.10⁻³, offrant des possibilités encore plus larges.

En résumé, il est proposé par l'invention d'utiliser pour matériau structurant un acier inox, un alliage de cobalt de la série MP35N, un métal précieux, du titane ou un alliage NiTi, de résistance à la fatigue élevée, pour former une structure multifilaire dont le diamètre *d* des brins n'excède pas 40 µm, cette dimension permettant en moyenne de garantir une résistance à la rupture en fatigue maximale dans les conditions extrêmes de contrainte auxquelles de telles structures peuvent être soumises.

On considèrera idéalement des brins de diamètre *d* compris entre 20 et 40 µm, des dimensions inférieures pouvant poser des problèmes de faisabilité technique et de coût.

Afin de garantir une visibilité aux rayons X suffisante pour l'implantation de la microsonde, il est nécessaire d'introduire le long du câble de coeur une quantité minimale de matériau radio-opaque.

La difficulté à cet égard est de concilier la résistance à la fatigue du câble avec la radio-opacité ainsi que la résistance à la corrosion. En effet, la plupart des matériaux utilisés pour leur visibilité aux rayons X, à savoir le tantale (Ta), le tungstène (W), l'iridium (Ir), le platine (Pt), l'or (Au) et leurs alliages, ne présentent pas en général une grande résistance à la fatigue. C'est pourquoi il y a avantage à adopter dans ce cas une structure de câble dite composite dans laquelle un matériau radio-opaque est ajouté au matériau structurant au sein du toron de brins unitaires.

Compte tenu de la sensibilité des équipements de radioscopie X actuels, on estime la présence minimale de matériau radio-opaque dans la structure composite à une surface de 0,008 mm² dans la section du câble de cœur, sans toutefois que la proportion de matériau radio-opaque ne dépasse 50%, ceci afin de ne pas dégrader les propriétés mécaniques des brins assurées par le matériau structurant.

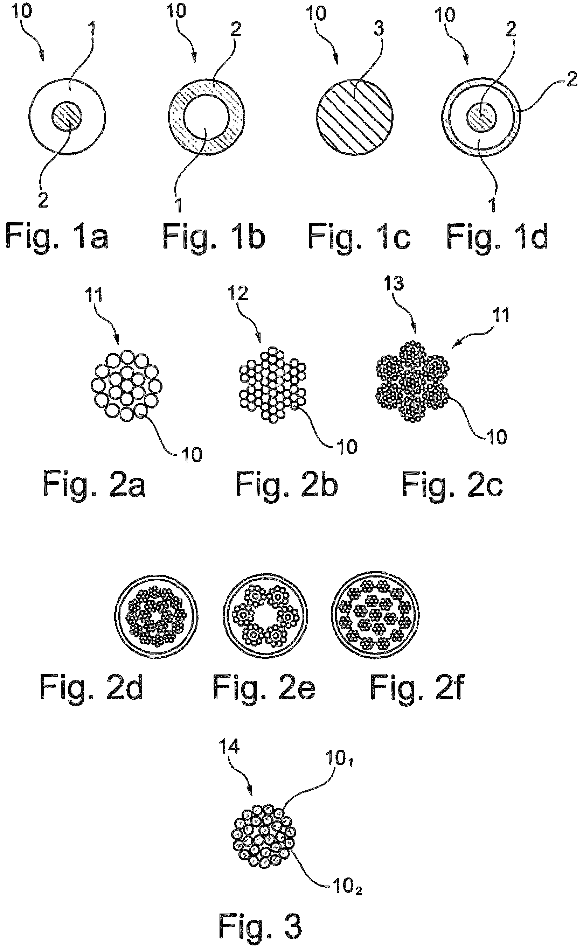

Comme le montrent les Figures 1a à 1d, la structure composite du câble de cœur est réalisée par des brins composites constitués, au moins, d'un matériau structurant 1 et d'un matériau radio-opaque 2. Plus précisément, la Figure 1a représente un brin dont le matériau structurant 1 se trouve à l'extérieur du brin et le matériau radio-opaque 2 à l'intérieur. A l'inverse, dans le brin de la Figure 1b, le matériau structurant 1 est à l'intérieur et le matériau radio-opaque 2 à l'extérieur. Le brin de la Figure 1c est réalisé avec un alliage 3 de matériau structurant et de matériau radio-opaque. Enfin, la structure du brin de la Figure 1d est plus complexe, avec deux sections extérieure et intérieure de matériau radio-opaque 2 entourant une section intermédiaire de matériau structurant 1.

Les brins 10 ainsi obtenus peuvent être toronnés pour constituer un câble de cœur pour la microsonde. Sur la Figure 2a est représenté un toron 11 de dix-neuf brins unitaires 10. Le toron 12 de la Figure 2b est formé par l'assemblage de sept groupes de sept brins 10. La Figure 2c montre un toron 13 de sept groupes de 19 brins assemblés selon le toron 11 de la Figure 2a. Enfin, des structures encore plus complexes sont illustrées sur les Figures 2d à 2f.

Selon la variante de la Figure 3, le câble 14 de cœur présente une structure composite réalisée non pas au niveau des brins mais au niveau du câble lui-même. Dans l'exemple proposé, des brins 10₁ constitués de matériau structurant entourent des brins 10₂ constitués de matériau radio-opaque.

S'agissant du nombre de brins par toron, on peut calculer que pour un diamètre de brins de 40 µm et une proportion de matériau radio-opaque de 50% occupant une section de 0,008 mm², le nombre total de brins tous matériaux confondus est de l'ordre de quinze brins. A l'inverse, pour un diamètre de brin de 15 µm et une proportion de matériau radio-opaque de 15% occupant la même section, le nombre total de brins atteint environ 300 brins.

Une autre caractéristique physique importante pour une microsonde est sa flexibilité. C'est cette propriété en effet qui permet au dispositif de stimulation de traverser les tortuosités de faible rayon et de garantir l'absence de traumaticité de la sonde en évitant de perforer les veines dans lesquelles elle circule. Pour garantir l'atraumaticité, l'extrémité de la microsonde sera arrondie en demi-sphère afin de minimiser ce risque de perforation.

En effectuant une comparaison avec les fils-guides existants utilisés dans les mêmes applications, la Demanderesse a pu établir qu'un diamètre externe du câble de cœur au plus égal à 0,50 mm permet d'obtenir un niveau suffisant de flexibilité et de compatibilité avec les tissus vivants.

D'une manière générale, la compatibilité des dispositifs implantables avec les techniques médicales modernes d'imagerie, telles que l'IRM, est fondamentale pour garantir un traitement optimal du patient.

En effet, du fait de sa structure globalement métallique, la microsonde présente un risque d'échauffement lié aux courants induits par "effet de peau" à l'extérieur des brins unitaires sous l'action du champ magnétique appliqué. Le faible diamètre donné aux brins est toutefois favorable à la dissipation thermique et réduit les effets d'échauffement dus à l'IRM. De plus, l'énergie thermique emmagasinée par les matériaux, déjà limitée en volume, peut encore être diminuée si les brins unitaires sont revêtus d'une couche extérieure en matériau à faible susceptibilité magnétique (la susceptibilité magnétique étant la faculté d'un matériau à s'aimanter sous l'action d'un champ magnétique extérieur).

Les matériaux les plus favorables dans cette application sont ceux dont la susceptibilité magnétique est inférieure à 2 000.10⁻¹² m³.mole⁻¹, notamment le tantale (Ta), le titane (Ti), le rhodium (Rh), le molybdène (Mo), le tungstène (W), le palladium (Pd), l'or (Au) et leurs alliages.

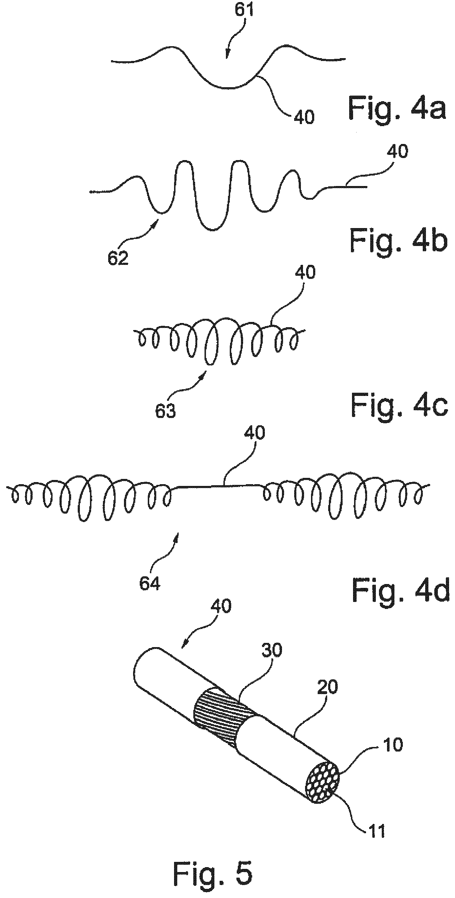

Concernant la transmission du courant électrique aux tissus, le concept retenu par l'invention est de ne pas prévoir d'électrodes rapportées, mais, comme le montre la Figure 5, d'utiliser le câble 11 de cœur conducteur lui-même pour former les électrodes en entourant partiellement le câble d'une couche 20 d'isolement en polymère. Une électrode 30 est donc constituée par une zone dénudée de câble. Cette technique de revêtement offre à l'électrode 30 un contact suffisant pour garantir la stimulation électrique des tissus.

La couche 20 d'isolement couvre l'ensemble de la structure conductrice du câble 11 de cœur, sauf au niveau des zones d'électrode réparties le long du microcâble ainsi réalisé.

De préférence, l'épaisseur de la couche 20 d'isolement n'excède pas 30% du diamètre extérieur du câble 11 de cœur, ceci pour éviter l'effet d'escalier en bordure d'électrode, l'isolant pouvant empêcher le contact de l'électrode avec le tissu.

Les caractéristiques requises pour la couche 20 d'isolement sont les suivantes :
- résistance à la fatigue,
- isolement électrique,
- biocompatibilité à long terme,
- biostabilité,
- possibilité de transformation et mise en œuvre compatible avec le conducteur du câble de cœur.

Les matériaux pouvant être utilisés dans ce cadre sont, par exemple :
- les polyuréthannes (PU),
- les polyesters (PET),
- les polyamides (PA),
- les polycarbonates (PC),
- les polyimides,
- les polymères fluorés,
- le polyéther-éther-kétone (PEEK),
- le poly-p-xylylène (parylène),
- le polyméthacrylate de méthyle (PMM).

Cependant, on privilégiera les matériaux à forte inertie chimique comme les polymères fluorés, qui présentent également une très bonne qualité d'isolation. Parmi ces composés, on peut citer :
- le PTFE (polytétrafluoroéthylène),
- le FEP (propylène perfluoré),
- le PFA (résine de copolymère perfluoroalkoxy),
- le THV (tétrafluoroéthylène, hexafluoropropylène, fluorure de vinylidène),
- le PVDF (polyfluorure de vinylidène),
- l'EFEP (éthylène propylène éthylène fluoré),
- l'ETFE (éthylène tétrafluoroéthylène).

Les procédés de réalisation de la couche d'isolement sur le câble de cœur sont multiples en fonction des matériaux utilisés :
- co-extrusion sur le conducteur, pour PU, PA, PEEK, polyimides et polymères fluorés ;
- dépôt par trempage dans une solution, pour PU, PA et polyimides ;
- échauffement d'un tube thermorétractable, pour PET et polymères fluorés ;
- dépôt chimique par voie gazeuse, pour le parylène ;
- traitements plasma pour améliorer l'adhésion entre les couches.

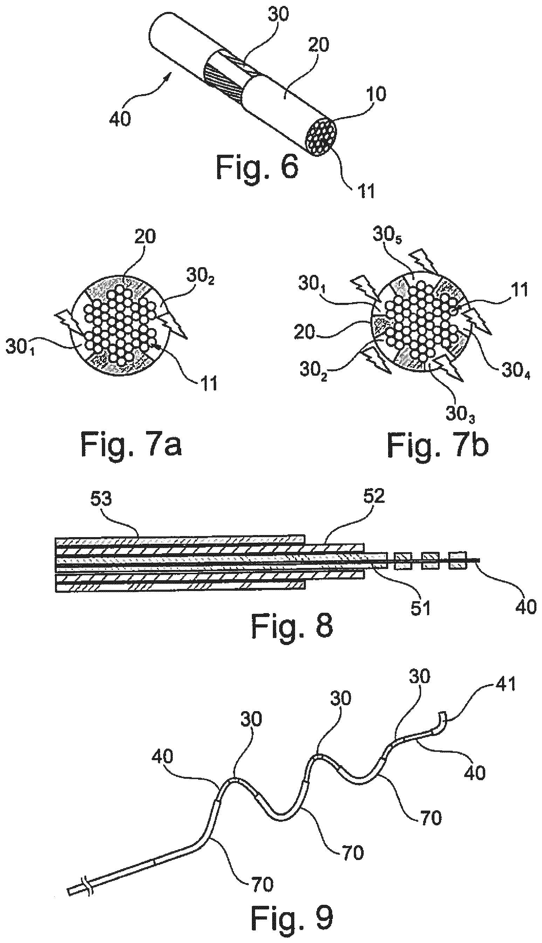

Lors de la mise en œuvre de ces procédés, les zones d'électrode peuvent être définies en déposant des couches d'isolement séparées les unes des autres, ou par dénudage partiel d'une couche d'isolement déposée sur la totalité du câble. Ce dénudage est effectué par exemple par ablation laser. Comme l'indiquent les Figures 6 et 7a, 7b, cette technique permet de réaliser des ouvertures partielles. En particulier, les Figures 7a et 7b montrent un microcâble avec respectivement deux zones dénudées 30₁, 30₂, et cinq zones dénudées 30₁, 30₂, 30₃, 30₄, 30₅.

Avantageusement, les zones d'électrode réparties le long du microcâble présentent une surface cumulée ne dépassant pas 20 mm², par exemple sous la forme de 40 électrodes de 0,5 mm² ou 20 électrodes de 1 mm², cette surface dépendant de l'application ainsi que des performances électriques de l'équipement associé.

Idéalement, afin de limiter la consommation de courant, il est cependant préférable de réaliser des électrodes d'une surface au plus égale à 0,5 mm², en augmentant ainsi la densité de courant locale.

Si cela s'avère nécessaire, le microcâble peut comporter au niveau des électrodes un renforcement de résistance à la corrosion, obtenu par ajout d'un revêtement dédié à très forte résistance. La résistance à la corrosion peut également résulter du choix d'une structure où le métal noble du matériau radio-opaque forme une couche extérieure gainant un coeur de matériau structurant.

Une première technologie utilisée en ce sens consiste en un dépôt submi-cronique (inférieur à 1 µm) par voie chimique ou électrochimique d'un matériau noble, du type de ceux mentionnés plus haut comme matériaux radio-opaques.

Une autre technique consiste à réaliser un tube composite de type DFT (*Drawn Filled Tube*), avec une couche supplémentaire de 1 à 2 µm de métal noble.

Une autre méthode encore consiste à réaliser un dépôt de carbone de type *Carbofilm* (marque déposée) permettant d'obtenir une protection anticorrosion et de bonnes performances en termes d'hémocompatibilité et de biocompatibilité.

Au besoin, la surface externe de la couche d'isolement proche des électrodes peut contenir un anti-inflammatoire de type stéroïde. Dans ce cas, une très fine couche de stéroïde est déposée en fin de processus de fabrication par un procédé de greffage chimique ou par réticulation de polymère, par exemple un polymère biodégradable de type PLAGA (acide polylactique co-glycolique) ou PLA (acide polylactique).

On peut également envisager que le produit anti-inflammatoire soit contenu dans le matériau constituant la couche d'isolement.

Enfin, la microsonde est terminée à partie proximale par un connecteur prévu pour être raccordé au générateur de l'appareil implantable.

Selon l'invention, du fait de la petite taille de la microsonde, il est envisagé de préformer celle-ci au niveau des électrodes afin de favoriser le contact électrique avec les tissus, et également afin de stabiliser mécaniquement la microsonde dans les vaisseaux.

Les préformes pourront être obtenues par formage de la partie métallique ou polymère de la sonde, par exemple par traitement thermique.

Les Figures 4a à 4d illustrent quelques modes de réalisation particuliers de la préforme : les préformes des Figures 4a et 4b ont une configuration plane en S, simple ou multiple, tandis que les préformes des Figures 4c et 4d sont configurées en spirale à trois dimensions, simple ou double.

Selon un autre aspect de l'invention, une caractéristique déterminante d'une microsonde est de pouvoir être aisément manipulable par le médecin lors de l'implantation.

Il est également important de minimiser les transitions de rigidité le long de la sonde de manière à réduire les concentrations de contrainte pouvant conduire à des fragilisations en fatigue du dispositif. Néanmoins, une certaine rigidification est nécessaire car une microsonde excessivement souple limiterait la manipulation en poussée.

La solution à ces difficultés consiste en une rigidification étagée, rendue possible par des moyens de dégressivité de rigidité ménagés entre la partie proximale et la partie distale de la microsonde. Il est alors possible de gérer le gradient de rigidité progressif le long de la sonde de manière à garantir, d'une part, une partie distale souple non traumatique permettant de passer au travers des tortuosités et, d'autre part, une partie proximale plus rigide permettant de transmettre la poussée exercée par le médecin au moyen de dispositifs appropriés.

Dans l'exemple proposé à la Figure 8, les moyens de dégressivité de rigidité sont réalisés par un empilement étagé de trois tubes 51, 52, 53 emboîtés les uns dans les autres sur le microcâble 40. Ces tubes, en PET (polyéthylène téréphtalate) par exemple, peuvent avoir une épaisseur de 5 à 20 µm.

Ainsi, la rigidité en extrémité proximale de la sonde peut être cinquante fois supérieure à la rigidité en extrémité distale, sans que cela nécessite d'ajouter de pièce mécanique supplémentaire. La robustesse de l'ensemble est également fortement accrue.

On peut aussi envisager des moyens de dégressivité de rigidité réalisés par une succession de tubes isodiamètre de rigidité décroissante, soudés entre eux. Toutefois, cette technique génère des risques de rupture au niveau des soudures entre les tubes.

Enfin, la Figure 9 illustre une autre variante consistant en un renforcement local de la microsonde par une succession de tubes 70 pour, indépendamment de l'isolation, conforter une préforme ou une angulation nécessaire à la fonction recherchée, en donnant à la microsonde une forme spécifique. On pourra également thermoformer l'extrémité 41 de la microsonde par ce type d'approche.

Cette structure pleine, sans anfractuosités, présente l'avantage important d'être plus facilement stérilisable en comparaison des sondes classiques. Sont ainsi réduits les risques de dégradation des matériaux de la microsonde dus à un procédé de stérilisation trop agressif.

On va maintenant décrire des exemples particuliers de réalisation d'une microsonde selon l'invention, destinés à être implantées dans différents sites du corps.

### Exemple 1

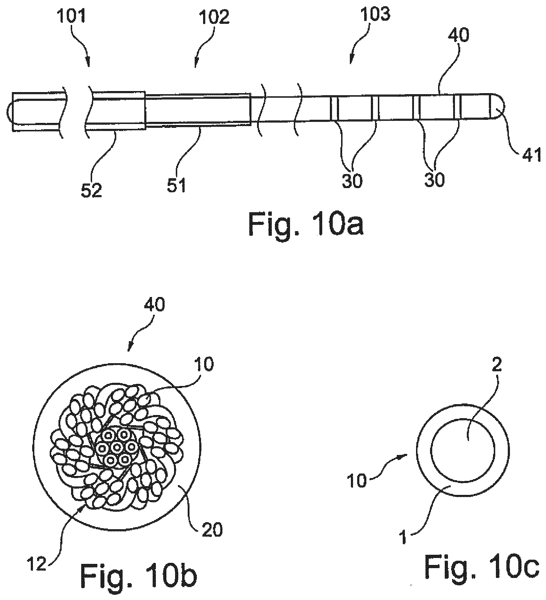

Les Figures 10a à 10e illustrent un exemple de réalisation d'une microsonde selon l'invention, destinée à être implantée dans une veine du sinus coronaire.

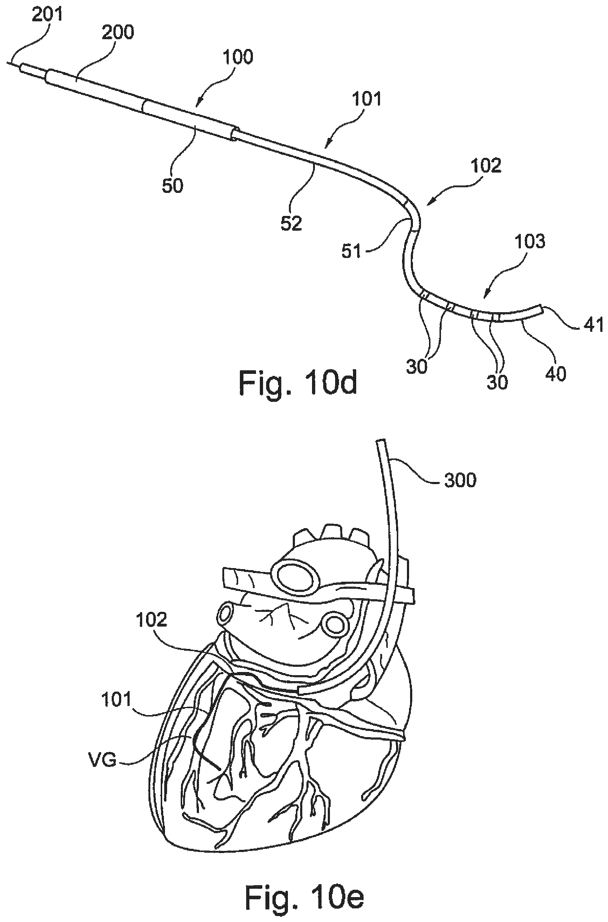

La microsonde représentée à la Figure 10a comprend un microcâble 40 dont une vue en coupe est montrée sur la Figure 10b. Le câble 12 de cœur du microcâble 40 est formé de brins unitaires 10 composites constitués, comme on peut le voir sur la Figure 10c, d'un noyau en métal structurant 1, ici un alliage MP35N sur un diamètre de 33 µm, et d'une enveloppe externe de 5 µm d'épaisseur de Pt/Ir 90/10 comme matériau radio-opaque 2. Le rapport entre les matériaux est de 75% pour le noyau et 25% pour l'enveloppe externe. Cette structure simple offre une bonne robustesse en fatigue ainsi qu'une résistance à la corrosion garantie par le platine.

Un câble 12 avec un cœur de 49 brins est nécessaire pour obtenir une surface apparente de platine de 0,011 mm², suffisante pour assurer une bonne visibilité sous radioscopie X. Le câble 12 de cœur conducteur a alors un diamètre de 0,30 mm, ce qui lui confère suffisamment de souplesse pour une utilisation intravasculaire, via le sinus coronaire par exemple.

Le câble 12 est recouvert d'une couche 20 d'isolement de type ETFE d'épaisseur 25 µm permettant une bonne isolation, compatible avec un procédé d'extrusion en ligne, pour un diamètre externe final de 0,35 mm dans la partie distale 103 de la microsonde.

Des ouvertures 30 formant électrodes sont réalisées par ablation laser dans la partie distale 103 sur une surface de 0,5 mm², permettant de réduire au maximum la consommation de courant.

Des tubes thermorétractables 51 et 52 en PET sont respectivement placés dans une zone intermédiaire 102 et en partie proximale 101, à 25 cm et 45 cm de la pointe distale 41 de la sonde, dont la longueur totale utile varie entre 90 et 120 cm.

La structure complète de la microsonde est donnée à la Figure 10d, sur laquelle on peut voir que la partie proximale 101 se termine par une zone 100 de transition formée par un tube 50 en polyuréthanne relié à un connecteur 200 de type IS-1 dont l'extrémité est munie d'une borne 201 de connexion électrique au générateur de l'équipement implantable.

Un procédé de mise en place d'une telle sonde, présenté sur la Figure 10e, consiste à placer la partie intermédiaire 102 dans le sinus coronaire et la partie distale 103 à électrodes multiples 30 dans les veines du réseau coronaire, pour stimuler ainsi le ventricule gauche VG. Cette opération est réalisée à l'aide d'un cathéter 300 de pose pouvant être retiré par découpe à l'aide d'un outil de coupe (*stitter*), comme pour la mise en place de sondes conventionnelles.

### Exemple 2

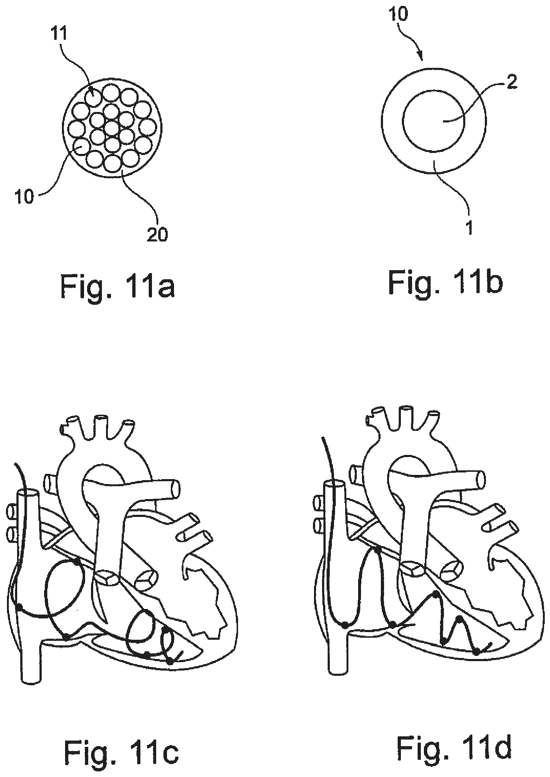

Un deuxième exemple de réalisation d'une microsonde destinée à être implantée dans une cavité cardiaque, par exemple une cavité droite, est illustré sur les Figures 11a à 11d.

Cette microsonde comprend un microcâble 40 dont une vue en coupe est montrée sur la Figure 11a. Le câble 11 de cœur du microcâble 40 est formé de brins unitaires 10 composites constitués, comme on peut le voir sur la Figure 11b, d'un noyau en tantale 2 en tant que matériau radio-opaque et d'une enveloppe externe 2 de matériau structurant, ici du nitinol. Le rapport entre les matériaux est de 25% de noyau et 75% pour l'enveloppe externe. Cette structure simple offre une élasticité externe ainsi qu'une bonne radio-opacité assurée par le noyau interne.

Il faut rappeler l'avantage du nitinol de présenter une mémoire de forme importante, particulièrement favorable au contact dans une cavité de grande taille.

Un câble de cœur 11 de dix-neuf brins est nécessaire pour obtenir une surface apparente de platine de 0,010 mm², suffisante pour assurer une bonne visibilité sous radioscopie X. Le câble de coeur 11 conducteur a alors un diamètre de 0,20 mm, ce qui lui confère suffisamment de souplesse pour une utilisation intra-cavitaire, ventricule et/ou oreillette droite en particulier.

Le câble 11 est recouvert d'une couche 20 d'isolement de type FEP d'épaisseur 25 µm permettant une bonne isolation et compatible avec un procédé d'extrusion en ligne, pour un diamètre externe final de 0,25 mm dans la partie distale de la microsonde.

Dans cet exemple de réalisation, il est possible d'utiliser une structure de renfort de type Polyimide ou PEEK de très faible épaisseur permettant de conserver les propriétés superélastiques du nitinol. On appliquera dans ce cas sur l'ensemble du microcâble un revêtement (*coating*) de type *Carbofilm* (marque déposée) présentant une résistance supérieure à la corrosion, ainsi qu'une biocompatibilité accrue. Ce type de revêtement, inférieur à 1 µm, permet de ne pas modifier les propriétés électriques de l'électrode tout en améliorant sensiblement les propriétés de compatibilité en surface avec le sang.

De plus, par un traitement approprié, il est possible de donner à la sonde une configuration lui permettant de se conformer à la cavité cardiaque en fonction des besoins de stimulation et d'anatomie associés. Les Figures 11c et 11d montrent deux conformations possibles de la sonde pour une stimulation des cavités droites.

### Exemple 3

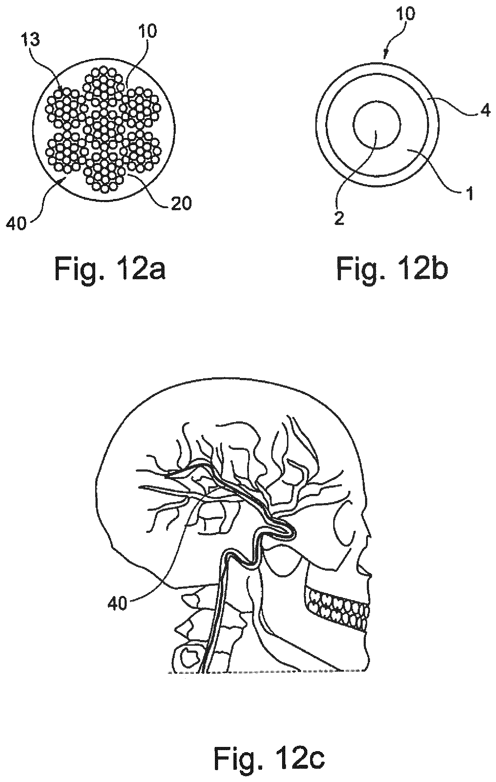

Un troisième exemple de réalisation d'une microsonde destinée à être implantée dans les cavités cérébrales est illustré sur les Figures 12a à 12c.

Dans cet exemple, une très bonne radio-opacité est nécessaire, ainsi qu'une grande souplesse et un diamètre très faible. D'autre part, pour ce type de produit, la compatibilité IRM est fondamentale.

Le microcâble 40 montré sur la Figure 12a comprend un câble 13 de cœur formé de brins unitaires composites 10 constitués, comme on peut le voir sur la Figure 12b, d'un fil tri-couche comprenant (i) un noyau 2 d'un matériau radio-opaque, ici du tantale, (ii) une couche intermédiaire 1 en titane comme matériau structurant, et (iii) une enveloppe externe 3 en palladium pour minimiser les effets de peau et obtenir une meilleure compatibilité structurelle avec l'IRM. Le rapport entre les trois matériaux est de 30% Ta / 65% Ti / 5% Pd. Cette structure, bien que moins résistante en fatigue au niveau du brin 10, est compensée par un diamètre unitaire de 16 µm, moins contraint mécaniquement.

Un câble 13 de cœur de 133 brins (7x19) est nécessaire pour obtenir une surface apparente de platine de 0,010 mm², suffisante pour assurer une bonne visibilité sous radioscopie X. Le câble de cœur conducteur 13 a alors un diamètre de 0,25 mm, très souple pour une utilisation intracérébrale.

Le câble 13 est recouvert d'une couche 20 d'isolement de type FEP, mécaniquement plus souple, d'épaisseur 25 µm permettant une bonne isolation et compatible avec un procédé d'extrusion en ligne, pour un diamètre externe final de 0,30 mm dans la partie distale de la microsonde.

Un polyéther bloc amide *Pebax* (marque déposée) peut être associé à la couche 20 d'isolement pour gérer les gradients de rigidité vers la partie proximale de la microsonde.

Un exemple d'implantation de cette sonde est montré sur la Figure 12c.

## Revendications

1. Une sonde de détection/stimulation destinée à être implantée dans des réseaux veineux, artériels ou lymphatiques, comportant un microcâble (40) de diamètre au plus égal à 2 French (0,66 mm), ledit microcâble (40) comprenant :
- un câble de cœur (11, 12, 13, 14) électriquement conducteur de diamètre au plus égal à 0,50 mm, formé par un toron d'une pluralité de brins (10) de diamètre unitaire (d) au plus égal à 40 µm, ledit câble de cœur comprenant un matériau structurant (1) présentant une résistance à la fatigue élevée ; et
- une couche d'isolement (20) en polymère entourant partiellement le câble de cœur sur une épaisseur au plus égale à 30% du diamètre du câble de cœur,
dans laquelle :
- ladite sonde est une microsonde constituée dans sa partie active, distale, par ledit microcâble (40) de diamètre au plus égal à 2 French (0,66 mm) ;
- ledit matériau structurant (1) du câble de cœur comprend un acier inox, un alliage de cobalt, un métal précieux, du titane ou un alliage NiTi ;
- le matériau radioopaque (2) est un matériau du groupe formé par : le tantale (Ta), le tungstène (W), l'iridium (Ir), le platine (Pt), l'or (Au) et leurs alliages ;
- des moyens de dégressivité de rigidité (51, 52, 53) sont ménagés le long de la microsonde entre sa partie proximale et sa partie distale,
**caractérisée en ce que** :
- le câble de cœur (11, 12, 13, 14) du microcâble possède une structure composite constituée à partir, au moins, dudit matériau structurant (1) et d'un matériau radioopaque (2) constituant au moins environ 0,008 mm² de la section du câble de cœur dans une proportion au plus égale à 50% ;
- au moins une zone dénudée (30) est ménagée dans la couche d'isolement (20) de manière à former au moins une électrode sur une surface totale cumulée au plus égale à 20 mm² ;
- la microsonde est conformée au niveau des électrodes (30) selon au moins une préforme (61, 62, 63, 64) de contact électrique et de stabilisation mécanique ; et
- la microsonde comprend des moyens de renforcement local incluant une succession de tubes (70) pour, indépendamment de l'isolation, conforter la préforme dudit microcâble (40).

2. La microsonde de la revendication 1, dans laquelle la structure composite du câble de cœur (11, 12, 13) est réalisée par des brins composites (10) constitués, au moins, d'un matériau structurant (1) et d'un matériau radio-opaque (2).

3. La microsonde de la revendication 2, dans laquelle :
- le matériau structurant (1) est à l'extérieur et le matériau radio-opaque (2) à l'intérieur du brin (10), ou
- le matériau structurant (1) est à l'intérieur et le matériau radio-opaque (2) à l'extérieur du brin (10), ou
- une section extérieure et une section interne de matériau radio opaque (2) entourent une section intermédiaire de matériau structurant (1).

4. La microsonde de la revendication 1, dans laquelle la structure composite du câble (14) de cœur est réalisée par des brins (10₁) constitués de matériau structurant (1) et des brins (10₂) constitués de matériau radio-opaque (2).

5. La microsonde de la revendication 1, dans laquelle le diamètre unitaire (d) des brins (10) est compris entre 20 et 40 µm.

6. La microsonde de la revendication 1, dans laquelle ladite pluralité de brins du toron comprend entre 15 et 300 brins.

7. La microsonde de la revendication 1, dans laquelle le polymère constituant la couche d'isolement (20) est un polymère fluoré.

8. La microsonde de la revendication 1, dans laquelle les brins (10) comprennent une couche extérieure en matériau (3) à faible susceptibilité magnétique, inférieure à 2000.10⁻² m³.mole⁻¹.

9. La microsonde de la revendication 8, dans laquelle le matériau (3) à faible susceptibilité magnétique est un matériau du groupe formé par : le tantale (Ta), le titane (Ti), le rhodium (Rh), le molybdène (Mo), le tungstène (W), le palladium (Pd), l'or (Au) et leurs alliages.

10. La microsonde de la revendication 1, dans laquelle la surface d'une électrode (30) est au plus égale à 0,5 mm².

11. La microsonde de la revendication 1, dans laquelle le microcâble (40) comporte au niveau des électrodes (30) un revêtement de résistance à la corrosion.

12. La microsonde de la revendication 1, dans laquelle la surface externe de la couche d'isolement (20) proche des électrodes (30) contient un anti-inflammatoire.

13. La microsonde de la revendication 1, dans laquelle le matériau constituant la couche d'isolement (20) contient un anti-inflammatoire.

14. La microsonde de la revendication 1, dans laquelle la préforme est :
- une préforme plane (61,62) en S, ou
- une préforme à trois dimensions (63, 64) en spirale.

15. La microsonde de la revendication 1, dans laquelle :
- les moyens de dégressivité de rigidité sont réalisés par un empilement étagé de tubes (51, 52, 53) emboîtés les uns dans les autres, ou
- les moyens de dégressivité de rigidité sont réalisés par une succession de tubes isodiamétres de rigidité décroissante.

## Patentansprüche

1. Detektions-/Stimulationssonde zur Implantation in venöse, arterielle oder lymphatische Systeme, die ein Mikrokabel (40) mit einem Durchmesser von höchstens gleich 2 French (0,66 mm) umfasst, wobei das Mikrokabel (40) Folgendes umfasst:
- ein elektrisch leitfähiges Kernkabel (11, 12, 13, 14) mit einem Durchmesser von höchstens gleich 0,50 mm, bestehend aus einer Litze aus einer Vielzahl von Fäden (10) mit einem Einheitsdurchmesser (d) von höchstens gleich 40 µm, wobei das Kernkabel ein Strukturmaterial (l) mit hoher Ermüdungsbeständigkeit umfasst; und
- eine Isolierschicht (20) aus Polymer, die das Kernkabel teilweise umgibt und deren Dicke höchstens gleich 30 % des Durchmessers des Kernkabels beträgt,
wobei:
- die genannte Sonde eine Mikrosonde ist, deren aktiver, distaler Teil aus dem genannten Mikrokabel (40) mit einem Durchmesser von höchstens gleich 2 French (0,66 mm) besteht;
- das genannte Strukturmaterial (1) des Kernkabels aus Edelstahl, einer Kobaltlegierung, einem Edelmetall, Titan oder einer NiTi-Legierung besteht;
- das röntgendichte Material (2) ein Material ist aus der Gruppe bestehend aus: Tantal (Ta), Wolfram (W), Iridium (Ir), Platin (Pt), Gold (Au) und deren Legierungen;
- entlang der Mikrosonde zwischen ihrem proximalen und ihrem distalen Abschnitt Mittel zur Verringerung der Steifigkeit (51, 52, 53) vorgesehen sind,
**dadurch gekennzeichnet, dass**:
- das Kernkabel (11, 12, 13, 14) des Mikrokabels eine Verbundstruktur aufweist, die mindestens aus dem genannten Strukturmaterial (1) und einem röntgendichten Material (2) besteht, das mindestens etwa 0,008 mm² des Querschnitts des Kernkabels ausmacht, wobei dessen Anteil höchstens gleich 50 % beträgt;
- in der Isolierschicht (20) mindestens ein freigelegter Bereich (30) ausgebildet, sodass mindestens eine Elektrode auf einer kumulierten Gesamtfläche von höchstens gleich 20 mm² ausgebildet wird;
- die Mikrosonde an den Elektroden (30) entsprechend mindestens einer Vorform (61, 62, 63, 64) für den elektrischen Kontakt und zur mechanischen Stabilisierung geformt ist; und
- die Mikrosonde lokale Verstärkungsvorrichtungen umfasst, darunter eine Abfolge von Rohren (70), um die Vorform des genannten Mikrokabels (40) unabhängig von der Isolierung zu verstärken.

2. Mikrosonde nach Anspruch 1, wobei die Verbundstruktur des Kernkabels (11, 12, 13) durch Verbundfäden (10) gebildet wird, die mindestens aus einem Strukturmaterial (1) und einem röntgendichten Material (2) bestehen.

3. Mikrosonde nach Anspruch 2, wobei:
- das Strukturmaterial (l) sich außerhalb und das röntgendichte Material (2) innerhalb des Fadens (10) befindet, oder
- das Strukturmaterial (l) sich innerhalb und das röntgendichte Material (2) außerhalb des Fadens (10) befindet, oder
- ein äußerer Abschnitt und ein innerer Abschnitt des röntgendichten Materials (2) einen mittleren Abschnitt des Strukturmaterials (l) umgeben.

4. Mikrosonde nach Anspruch 1, wobei die Verbundstruktur des Kernkabels (14) aus Fäden (10₁) aus Strukturmaterial (1) und Fäden (10₂) aus röntgendichtem Material (2) besteht.

5. Mikrosonde nach Anspruch 1, wobei der Einheitsdurchmesser (d) der Fäden (10) zwischen 20 und 40 µm liegt.

6. Mikrosonde nach Anspruch 1, wobei die Vielzahl von Fäden der Litze zwischen 15 und 300 Fäden umfasst.

7. Mikrosonde nach Anspruch 1, wobei das die Isolierschicht (20) bildende Polymer ein fluoriertes Polymer ist.

8. Mikrosonde nach Anspruch 1, wobei die Fäden (10) eine Außenschicht aus einem Material (3) mit einer geringen magnetischen Suszeptibilität von weniger als 2000·10⁻² m³·mol⁻¹ aufweisen.

9. Mikrosonde nach Anspruch 8, wobei das Material (3) mit geringer magnetischer Suszeptibilität ein Material aus der Gruppe ist bestehend aus: Tantal (Ta), Titan (Ti), Rhodium (Rh), Molybdän (Mo), Wolfram (W), Palladium (Pd), Gold (Au) und deren Legierungen.

10. Mikrosonde nach Anspruch 1, wobei die Fläche einer Elektrode (30) höchstens gleich 0,5 mm² beträgt.

11. Mikrosonde nach Anspruch 1, wobei das Mikrokabel (40) an den Elektroden (30) eine korrosionsbeständige Beschichtung aufweist.

12. Mikrosonde nach Anspruch 1, wobei die äußere Fläche der Isolierschicht (20) in der Nähe der Elektroden (30) ein entzündungshemmendes Mittel enthält.

13. Mikrosonde nach Anspruch 1, wobei das Material, aus dem die Isolierschicht (20) besteht, ein entzündungshemmendes Mittel enthält.

14. Mikrosonde nach Anspruch 1, wobei die Vorform Folgendes ist:
- eine flache S-förmige Vorform (61, 62), oder
- eine dreidimensionale, spiralförmige Vorform (63, 64).

15. Mikrosonde nach Anspruch 1, wobei:
- die Mittel zur Verringerung der Steifigkeit durch eine stufenweise Anordnung von ineinander gesteckten Rohren (51, 52, 53) realisiert werden, oder
- die Mittel zur Verringerung der Steifigkeit durch eine Abfolge von Rohren mit gleichem Durchmesser und abnehmender Steifigkeit realisiert werden.

## Claims

1. Detection/stimulation probe intended for implantation in venous, arterial or lymphatic networks, comprising a microcable (40) with a diameter of at most 2 French (0.66mm), said microcable (40) comprising:
- an electrically conductive core cable (11, 12, 13, 14) with a diameter of at most 0.50mm, formed by a strand comprising a plurality of wires (10) with a unit diameter (d) of at most 40µm, said core cable comprising a structuring material (l) having a high fatigue resistance; and
- an insulating layer (20) made of polymer, partially surrounding the core cable to a thickness of at most 30% of the core cable's diameter,
wherein:
- said probe is a microprobe comprising, in its active, distal part, the said microcable (40) with a diameter of at most 2 French (0.66mm);
- said structuring material (1) of the core cable comprises stainless steel, a cobalt alloy, a precious metal, titanium or a NiTi alloy;
- the radiopaque material (2) is a material selected from the group consisting of: tantalum (Ta), tungsten (W), iridium (Ir), platinum (Pt), gold (Au) and their alloys;
- means for reducing stiffness (51, 52, 53) are provided along the length of the microprobe between its proximal end and its distal end,
**characterised in that**:
- the core cable (11, 12, 13, 14) of the microcable has a composite structure comprising, at least, said structuring material (1) and a radiopaque material (2) constituting at least approximately 0.008mm² of the section of the core cable, in a proportion not exceeding 50%;
- at least one exposed area (30) is formed in the insulating layer (20) so as to form at least one electrode on a total cumulative surface area of no more than 20mm²;
- the microprobe is shaped at the electrodes (30) in accordance with at least one preform (61, 62, 63, 64) for electrical contact and mechanical stabilisation; and
- the microprobe comprises local reinforcement means, including a series of tubes (70), to reinforce the preform of the microcable (40), independently of the insulation.

2. Microprobe of claim 1, wherein the composite structure of the core cable (11, 12, 13) is formed by composite wires (10) comprising, at least, a structuring material (1) and a radiopaque material (2).

3. Microprobe of to claim 2, wherein:
- the structuring material (l) is on the outside and the radiopaque material (2) is on the inside of the wire (10), or
- the structuring material (l) is on the inside and the radiopaque material (2) is on the outside of the wire (10), or
- an outer section and an inner section of radiopaque material (2) surround an intermediate section of structuring material (1).

4. Microprobe of claim 1, wherein the composite structure of the core cable (14) is formed by wires (10₁) made of structuring material (1) and wires (10₂) made of radiopaque material (2).

5. Microprobe of claim 1, wherein the unit diameter (d) of the wires (10) is between 20 and 40µm.

6. Microprobe of claim 1, wherein the said plurality of wires of the strand comprises between 15 and 300 wires.

7. Microprobe of claim 1, wherein the polymer forming the insulating layer (20) is a fluorinated polymer.

8. Microprobe of claim 1, wherein the strands (10) comprise an outer layer of material (3) having a low magnetic susceptibility of less than 2000.10⁻² m³.mol⁻¹.

9. Microprobe of claim 8, wherein the material (3) having a low magnetic susceptibility is a material selected from the group consisting of: tantalum (Ta), titanium (Ti), rhodium (Rh), molybdenum (Mo), tungsten (W), palladium (Pd), gold (Au) and alloys thereof.

10. Microprobe of claim 1, wherein the surface area of an electrode (30) is at most 0.5mm².

11. Microprobe of claim 1, wherein the microcable (40) comprises a corrosion-resistant coating at the electrodes (30).

12. Microprobe of claim 1, wherein the outer surface of the insulating layer (20) near the electrodes (30) contains an anti-inflammatory agent.

13. Microprobe of claim 1, wherein the material constituting the insulating layer (20) contains an anti-inflammatory agent.

14. Microprobe of claim 1, wherein the preform is:
- a flat S-shaped preform (61, 62), or
- a three-dimensional spiral preform (63, 64).

15. Microprobe of claim 1, wherein:
- the means for reducing stiffness are provided by a stepped stack of tubes (51, 52, 53) nested one inside the other, or
- the means for reducing stiffness are provided by a series of tubes of the same diameter, with decreasing stiffness.
